(19) Europäisches Patentamt / European Patent Office / Office européen des brevets

(11) **EP 4 304 705 B1**

(12) **EUROPEAN PATENT SPECIFICATION**

(45) Date of publication and mention
of the grant of the patent:
**06.05.2026 Bulletin 2026/19**

(21) Application number: **22713213.1**

(22) Date of filing: **10.03.2022**

(51) International Patent Classification (IPC):
*A61N 1/36* (2006.01)    *A61B 5/316* (2021.01)
*A61B 5/00* (2006.01)    *A61B 5/383* (2021.01)
*A61B 5/388* (2021.01)

(52) Cooperative Patent Classification (CPC):
**A61N 1/36014; A61B 5/383; A61B 5/388;**
**A61B 5/4041; A61B 5/4836; A61B 5/7267;**
A61B 5/6814; A61B 5/6822; A61B 5/6823;
A61B 5/7246

(86) International application number:
**PCT/US2022/019798**

(87) International publication number:
**WO 2022/192569 (15.09.2022 Gazette 2022/37)**

(54) **STIMULATION SYSTEM**

STIMULATIONSSYSTEM

SYSTÈME DE STIMULATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2021 US 202163160605 P**

(43) Date of publication of application:
**17.01.2024 Bulletin 2024/03**

(73) Proprietor: **Alphatec Spine, Inc.**
**Carlsbad, CA 92008 (US)**

(72) Inventors:
• **CLEVELAND, Daniel, Milton**
**Carlsbad, CA 92008 (US)**
• **JOHNS, Gregg**
**Carlsbad, CA 92008 (US)**
• **SNOW, Robert, Gerard**
**Carlsbad, CA 92008 (US)**
• **O'BRIEN, Richard, Arthur**
**Carlsbad, CA 92008 (US)**

(74) Representative: **Morbidini, Marco**
**Kilburn & Strode LLP**
**Lacon London**
**84 Theobalds Road**
**London WC1X 8NL (GB)**

(56) References cited:
US-A1- 2010 312 124    US-A1- 2014 324 118
US-A1- 2018 078 210    US-A1- 2018 140 843
US-A1- 2018 310 849    US-A1- 2018 360 336
US-A1- 2020 315 478

EP 4 304 705 B1

## Description

### CROSS-REFERENCE TO RELATED APPLICATIONS

The present application claims priority to U.S. Provisional App. No. 63/160,605, filed March 12, 2021, and entitled "Stimulation System".

### TECHNICAL FIELD

The subject matter described herein relates generally to patient monitoring and clinical neurophysiology, and more specifically to a stimulation system for detecting and identifying patient physiological responses.

### BACKGROUND

Monitoring patients by recording waveforms in response to stimulation delivered to the patients during surgery allows for early identification and prevention of impending injuries, such as a nerve injury. Generally, highly trained technologists under physician supervision may monitor patients during surgery using sophisticated, multichannel amplifiers and display equipment. Unfortunately, such personnel and equipment are costly and may be limited in their availability and/or require pre-booking. Such personnel may also subjectively analyze the waveforms during stressful circumstances, decreasing the accuracy, speed, and efficiency in detecting physiological responses, and thus leading to an increase in the risk of injury caused to patients during surgery. Detection of patient physiological responses and changes in the responses within the recorded waveforms may also be difficult, as such responses may be small, and the waveforms may include a large amount of ongoing noise signals. Thus, it may be difficult for technologists to recognize the patient physiological responses and to properly alert for changes in the patient physiological responses. This may lead to an increase in injuries caused to the patient during surgery. US 2014/0324118 A1 discloses devices and methods for treating medical disorders with evoked potentials and vagus nerve stimulation.

### SUMMARY

Systems, methods, and articles of manufacture, including computer program products, are provided by the present disclosure for configuring a medical device and recommending medication dosages based at least in part on a health condition of a patient. For example, the system may provide more accurate medication preparation and delivery configurations and/or dosages for a patient to more effectively, efficiently, and quickly treat the patient.
The invention is defined by claim 1.
Additionally, a non-claimed method for detecting and identifying a patient physiological response is provided. The method may include stimulating, via a stimulating electrode coupled to a patient, one or more nerves of the patient. The method may also include recording, via a recording electrode coupled to the patient, a plurality of resultant electrical waveforms. The method may also include determining, based on the plurality of resultant electrical waveforms, whether at least a subset of the plurality of resultant electrical waveforms includes a patient physiological response. The determining may include comparing the subset of resultant electrical waveforms of the plurality of resultant electrical waveforms to a model waveform derived-sometimes in real-time-from a database of a plurality of template waveforms. The determining may also include determining, based on the comparison, one or more comparison features. The comparison feature(s) may indicate whether the patient physiological response exists in the subset of resultant electrical waveforms. The method may further include displaying, via a display, an indication that the patient physiological response exists in the subset of resultant electrical waveforms.
In some aspects, the model waveform includes a predicted physiological response and a plurality of artifact signals.
In some aspects, the comparison feature(s) further indicate(s) whether an artifact signal exists in the subset of resultant electrical waveforms.
In some aspects, the method also includes displaying, via the display, an indication that the artifact signal exists in the subset of resultant electrical waveforms.
In some aspects, the determining whether at least the subset of the plurality of resultant electrical waveforms includes the patient physiological response further includes labeling, based on the comparison feature(s), the subset of the plurality of resultant electrical waveforms with a positive label or a negative label. The positive label indicates that the patient physiological response exists and the negative label indicates that the patient physiological response does not exist.
In some aspects, the determination of whether the plurality of resultant electrical waveforms includes the patient physiological response further includes: determining that the patient physiological response exists when the comparison feature(s) of the subset of electrical waveforms is within a threshold range of a threshold value of the comparison feature(s).

In some aspects, the comparison feature(s) include(s) one or more of a means squared error between the subset of resultant electrical waveforms and the model waveform, a correlation between the subset of resultant electrical waveforms and the model waveform, a physiological coefficient amplitude, a power of fundamental harmonic noise, a THP of higher harmonic noise, a ratio of a physiological coefficient of the subset of resultant electrical waveforms to a power of the harmonic noise, and a ratio of variance of the patient physiological response of the subset of resultant electrical waveforms to the predicted physiological response of the model.

In some aspects, the determination of whether the plurality of resultant electrical waveforms includes the patient physiological response further includes: determining that the patient physiological response exists when a polarity of each electrical waveform of the subset of resultant electrical waveforms are the same.

In some aspects, the comparison feature represents a comparison of a morphology of the subset of resultant electrical waveforms to a morphology of the model waveform.

In some aspects, the comparison feature includes a plurality of comparison features, and the labeling is further based on a mathematical representation of the plurality of comparison features.

In some aspects, the labeling includes comparing the comparison feature to a plurality of previously generated comparison features.

In some aspects, the subset of the plurality of resultant electrical waveforms are time-locked.

In some aspects, the model waveform is generated using a joint fast orthogonal search method. The joint fast orthogonal search method may generate the model waveform using the equation:

$$\bar{Y}(n) = C_0 + C_1 X_1(n) + \sum_{i=1}^{N} a_i \, sin(\omega n + \phi)$$

where $C_0$ is a coefficient, $X_1(n)$ is a physiological template, $a_i = \sqrt{C_2^2 + C_3^2}$, $\omega$ is a noise frequency, and $\phi$ is a phase.

In some aspects, the stimulating includes transmitting a plurality of electrical stimuli. The stimulating electrode is in communication with an evoked potential detection device configured to monitor the one or more peripheral nerves of the patient.

In some aspects, the plurality of resultant electrical waveforms is received by the evoked potential detection device. The resultant electrical waveforms may be generated by the patient in response to the electrical stimuli.

Without being claimed as such, implementations of the current subject matter can include methods consistent with the descriptions provided herein as well as articles that comprise a tangibly embodied machine-readable medium operable to cause one or more machines (e.g., computers, etc.) to result in operations implementing -- or signaling the need to implement -- one or more of the described features. Similarly, computer systems are also described that may include one or more processors and one or more memories coupled to the one or more processors. A memory, which can include a non-transitory computer-readable or machine-readable storage medium, may include, encode, store, or the like one or more programs that cause one or more processors to perform one or more of the operations described herein. Computer implemented methods consistent with one or more implementations of the current subject matter can be implemented by one or more data processors residing in a single computing system or multiple computing systems. Such multiple computing systems can be connected and can exchange data and/or commands or other instructions or the like via one or more connections, including, for example, to a connection over a network (e.g. the Internet, a wireless wide area network, a local area network, a wide area network, a wired network, or the like), via a direct connection between one or more of the multiple computing systems, etc.

The details of one or more variations of the subject matter described herein are set forth in the accompanying drawings and the description below. Other features and advantages of the subject matter described herein will be apparent from the description and drawings, and from the claims. The claims that follow this disclosure are intended to define the scope of the protected subject matter.

## DESCRIPTION OF DRAWINGS

The accompanying drawings, which are incorporated in and constitute a part of this specification, show certain aspects of the subject matter disclosed herein and, together with the description, help explain some of the principles associated with the disclosed implementations. In the drawings,

FIG. 1 depicts a system diagram illustrating a stimulation system, consistent with implementations of the current subject matter;

FIG. 2 depicts a functional block diagram of a system for monitoring nerve function, consistent with implementations of the current subject matter;

FIG. 3 depicts an example user interface, consistent with implementations of the current subject matter;

FIG. 4 depicts an example user interface, consistent with implementations of the current subject matter;

FIG. 5 depicts an example user interface, consistent with implementations of the current subject matter;

FIG. 6 depicts an example user interface, consistent with implementations of the current subject matter;

FIG. 7 depicts an example user interface, consistent with implementations of the current subject matter;

FIG. 8 depicts an example user interface, consistent with implementations of the current subject matter;

FIG. 9 depicts an example user interface, consistent with implementations of the current subject matter;

FIG. 10 depicts a flowchart illustrating a process for identifying and labeling a patient physiological response, consistent with implementations of the current subject matter;

FIG. 11 depicts a flowchart illustrating a process for generating physiological templates, consistent with implementations of the current subject matter;

FIG. 12 depicts a flowchart illustrating a process for generating a model waveform, consistent with implementations of the current subject matter;

FIG. 13 depicts a flowchart illustrating a process for identifying and labeling a patient physiological response, consistent with implementations of the current subject; and

FIG. 14 depicts a block diagram illustrating a computing system, in accordance with some example implementations.

When practical, similar reference numbers denote similar structures, features, or elements.

## DETAILED DESCRIPTION

Monitoring patients by recording waveforms or signals in response to stimulation delivered to the patients during surgery allows for early identification and prevention of impending injuries, such as a nerve injury. These waveforms may be produced by the patients in response to stimulation of a nerve, such as a peripheral nerve or other parts of the patient's nervous system.

Monitoring and identifying patient physiological responses from within the recorded waveforms may be fraught with difficulties due to the small size of the patient physiological responses, such as evoked potentials, and the large amounts of ongoing noise or artifact signals. This makes recognizing the physiological responses, and determining when to alert for these responses difficult, especially in stressful situations during surgery. In some systems, alerts are generated automatically, but substantial noise and variability can cause false alerts. For example, the generated signals may be preprocessed and/or may not accurately measure or account for noise. In other systems, highly trained technologists, such as trained technologists under physician supervision, may monitor patients during surgery using sophisticated, multichannel amplifiers and display equipment. Such personnel and equipment are costly and may be limited in their availability or require pre-booking. The personnel may also subjectively analyze the waveforms under stressful situations, decreasing the accuracy, speed, and efficiency in detecting physiological responses, and thus leading to an increase in injuries caused to the patient during surgery. Subjectively monitoring the signals in real-time may lead to false alerts, and/or inaccurately identifying a patient physiological response may lead to an increased risk of injuries caused to the patient during surgery.

The stimulation system described herein may accurately and automatically detect and/or identify a patient physiological response, such as an electrophysical evoked potential. The stimulation system described herein may accurately detect a patient physiological response by, for example, comparing a subset of the recorded waveforms to a model waveform generated by the system and stored in a database of model waveforms. Thus, the stimulation system described herein may automatically and more accurately identify, in real-time, the patient physiological response from recorded waveforms that also include unwanted artifact and noise signals. In some implementations, the stimulation system may detect the physiological response without a technologist or other personnel monitoring the recorded signals or subjectively assessing, in real-time, the recorded waveforms. Thus, the stimulation system described herein may reduce erroneous assessments of signals during surgery, reduce the risk of causing an injury to the patient, reduce errors in the assessment of recorded waveforms due to noise or artifact signals, and/or the like. The stimulation system described herein may be implemented as part of and/or in conjunction with a response identification device. The stimulation system described herein may additionally and/or alternatively be used during any surgery or situation where a patient is at risk, to detect and/or identify a patient physiological response, indicate whether the patient physiological response is found, and ameliorate positioning effect or other nerve injury or abnormality.

As noted above, electrical noise interference or artifact signals, which may result from electrical noise generators such as power cables, patient warming devices, and other electronic surgical instrumentation, placement of electrodes, movement of the patient, and/or the like, may significantly alter the recorded electrical waveforms during surgery. Since the recorded patient physiological response signals, such as the evoked potentials, may be particularly small, even a few aberrant waveforms heavily affected by noise can markedly change the apparent amplitude (height) or latency (time of onset) of a waveform of interest. In some instances, noise or artifact signals may be at least partly avoided by careful choice of stimulation frequencies and filtering the waveforms. For example, such methods may work in several ways: (1) by limiting recorded waveform frequency range; (2) by rejecting periods of recordings where signals of high amplitude that contain clear artifacts are present; and (3) by extending the number of averages included in an averaged signal. Yet, standard filters (e.g., frequency filters, rejection threshold filters, etc.) that limit the frequency range of the recordings or waveform classifiers that reject raw recordings over a certain amplitude threshold may not remove sufficient noise or artifact signals from evoked potential recordings, leading to an inability to record accurate signals and thus, may result in inaccurate detection of the physiological responses. Furthermore, these methods may be insufficient, as the physiological responses may fall within a frequency range of the noise and the noise may vary in frequency. As a result, the noise signals make it difficult for even trained and highly skilled technologists to interpret recorded waveforms during surgery leading to erroneous assessments and identifications of physiological responses, and in turn, injury to the patient.

The stimulation system described herein may accurately detect a physiological response by, for example, comparing a subset of the recorded waveforms to a model waveform generated by the system and stored in a database of model waveforms. Thus, the system may automatically and more accurately identify, in real-time, the physiological response from waveforms that also include unwanted artifact and noise signals without significantly altering the character of the recorded waveforms. Additionally and/or alternatively, the stimulation system described herein generates alerts regarding the identification of the physiological responses that are consistent and accurate. Such configurations allow for automated determination of the alerts, and/or more accurate automated calculation and indication of the alerts. Accordingly, the system described herein may identify physiological responses and generate alerts free from subjective analysis of technologists during surgery and from the influence of variable noise and bias, while minimizing or eliminating false negative and false positive errors.

As described herein, the stimulation system may identify one or more patient physiological responses from recorded waveforms. The patient physiological responses may include one or more evoked potentials (EPs), such as somatosensory evoked potentials (SSEPs), auditory evoked potentials (AERs), motor evoked potentials (MEPs), brain stem auditory evoked potentials (BAEPs), and/or visual evoked potentials (VERs), among others. SSEPs may include the electrical signals generated by a patient's nervous system in response to an electrical stimulus applied to a peripheral nerve of the patient. EPs may include any potential recorded from the nervous system, resulting from the application of a stimulus to a portion of the patient's body. For example, an EP may include a voltage versus time signal obtained by ensemble averaging the electrophysiological responses to repetitive stimulation of a specific sensory neural system detected using suitable electrodes.

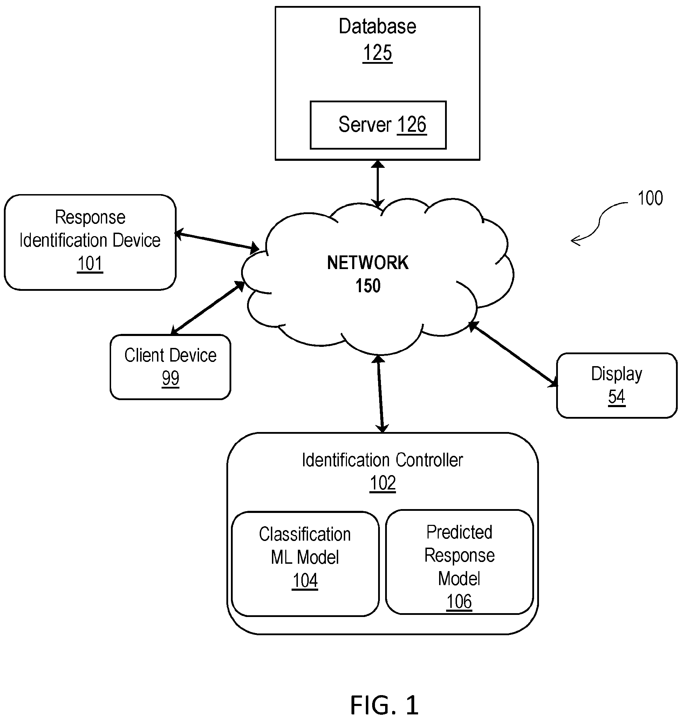

FIG. 1 depicts a system diagram illustrating a stimulation system 100, in accordance with some example implementations. Referring to FIG. 1, the stimulation system 100 may include a display 54, a client device 99, an identification controller 102, and/or a database 125. In some example implementations, the display 54, the client device 99, the identification controller 102, and/or the database 125 may form a portion of a response identification device 101 and/or may be positioned within a housing of the response identification device 101.

Referring to FIG. 1, the response identification device 101, the display 54, the client device 99, the identification controller 102, and/or the database 125 may be communicatively coupled via a network 150 and/or via a direct device-device connection as described herein. The network 150 may be a wired and/or wireless network including, for example, a public land mobile network (PLMN), a local area network (LAN), a virtual local area network (VLAN), a wide area network (WAN), the Internet, a short range radio connection, for example Bluetooth, a peer-to-peer mesh network, and/or the like.

The client device 99 may be a mobile device such as, for example, a smartphone, a tablet computer, a wearable apparatus, and/or the like. However, it should be appreciated that the client device 99 may be any processor-based device including, for example, a desktop computer, a laptop or mobile computer, a workstation, and/or the like. For example, via the client device 99, the clinician may be able to configure certain parameters of the response identification device 101, such as a stimulation sequence or stimulation strength, a response recording protocol, and the like. In some implementations, the client device 99 forms a part of the response identification device 101. Additionally, in some examples, via the client device 99, the user may configure various stimulation or protocols, and/or the like.

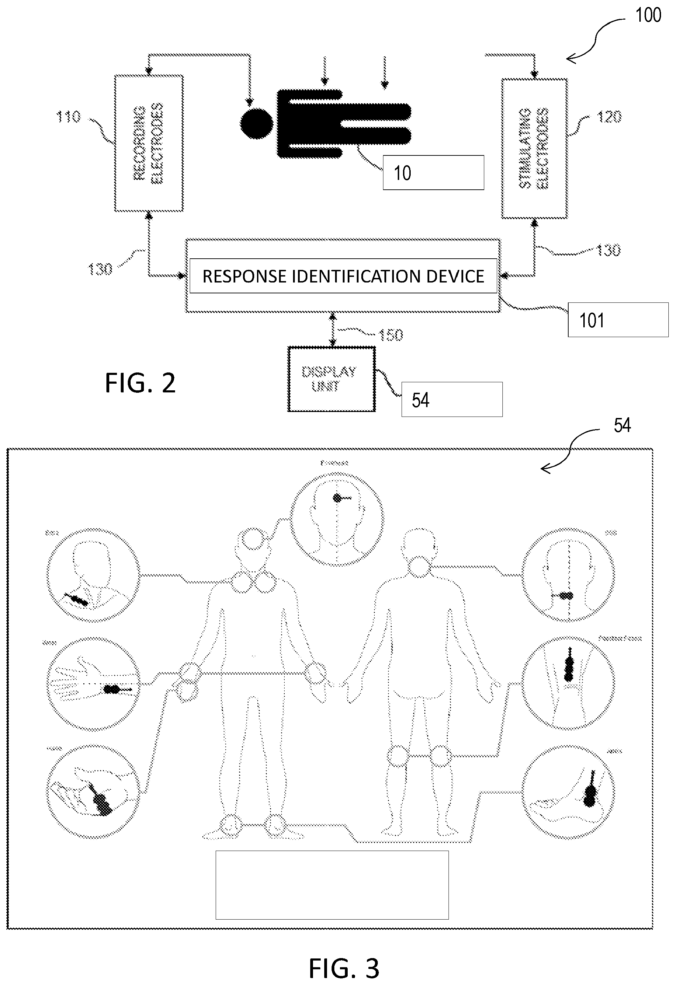

Referring to FIG. 2, the stimulation system 100 may include the response detection device 101, one or more recording electrodes 110 and/or one or more stimulating electrodes 120 coupled to a patient 10, and the display 54.

The stimulating electrodes 120 may be positioned on or near the arms or legs of the patient 10 over peripheral nervous structures such as, the ulnar nerves, median nerves, peroneal nerves, saphenous nerves, and/or posterior tibial nerves. The stimulating electrodes 120 may be intended for placement on a patient's skin on the wrists and/or ankles so that the electrodes are located over or near the ulnar nerves and posterior tibial nerves. These configurations allow for full patient monitoring of peripheral nerves, such as monitoring of the nerves in all limbs of the patient 10. In some implementations, the stimulation system 100 may be used for upper limb monitoring. In such implementations, the stimulating electrodes 120 may be intended for placement on the skin of a patient's wrists, for example, over or near the ulnar nerves.

The recording electrodes 110 may be positioned over the trunk, spine, neck, and/or head of the patient 10. The recording electrodes 110 are intended to be placed on the skin on or over the cervical vertebra 5 (C5) just below the hairline, the forehead, the left and right Erb's points near the clavicle, and/or the left and right Popliteal Fossa just above the knee, of the patient 10.

As shown in FIG. 2, the response identification device 101 may be coupled to the recording electrodes 110 and the stimulating electrodes 120, such as via a plurality of cables 130. The response identification device 101 may also be electrically, electronically, and/or mechanically coupled to the display 52, such as via a link 150. The link 150 may include internal wiring and/or an external cable. In some implementations, the link 150 is a wireless communication link. For example, the response identification device 101 may be wirelessly coupled to the display 52 via Bluetooth® or other radiofrequency signal or via near field communications or a cellular signal.

The response identification device 101 may apply electrical stimulation to peripheral nerves of a patient by sending electrical signals to the stimulating electrodes 120 located on some or all of a patient's limbs. Repeated stimulation elicits a response of the patient's nervous system in the form of physiological responses, such as EPs, which travel up the peripheral nerves, through the dorsal column of the spinal cord, and to the brain. EPs may be detected and changes in the monitored EP may indicate changes in nerve function. For example, the recording electrodes 110 may receive one or more resultant electrical waveforms in response to stimulation being provided to the patient 10 via the stimulating electrodes 120. The response identification device 101 may detect changes, such as changes in latency, changes in amplitude, or changes in morphology, in the EPs. Based on the observed changes, the response identification device 101 may identify potential injuries caused by a physical position of the patient's body, the stimulation being delivered to the patient, and/or the like. In some implementations, the response identification device 101 identifies a particular nerve structure or body region affected by positioning effect or the stimulation based on the EPs. The response identification device 101 may additionally and/or alternatively recommend actions, such as via the display 54, to ameliorate the injuries by recommending changes in position.

As noted above, the stimulation system 100 may include one or more stimulating electrodes 120. The response identification device 101 may sequentially stimulate peripheral nerves of the patient 10 via the stimulating electrodes 120 while recording the EPs via the recording electrodes 110. Thus, in some implementations, the stimulating electrodes 120 are coupled to the response identification device 101 as an output, and the recording electrodes 110 are coupled to the response identification device 101 as an input.

The response identification device 101 may include various circuitry components, such as electric stimulators, pre-amplifiers, amplifiers and/or other components, to control stimulation and process the return signals. In some implementations, the response identification device 101 may average together the response to several stimuli to reduce noise in the signal.

As described herein, the response identification device 101, such as via the response identification controller 102, may analyze signals and determine when warnings and alerts are appropriate. For example, the response identification device 101 may send signals to the display 54 to display warnings and/or alerts, such as when the stimulation is approaching a nerve of the patient.

The display 54 may form a part of the response identification device 101 and/or the client device 99, and/or may be separately coupled to the response identification device 101 and/or the client device 99. The display 54 may also include a user interface. The user interface may form a part of a display screen of the display 54 that presents information to the user (e.g., a clinician, a patient, a technologist, and/or the like) and/or the user interface may be separate from the display screen. For example, the user interface may include one or more buttons, or portions of the display screen that is configured to receive an entry from the user.

The display 54 may display various information, such as biographical information of a patient, suggested locations of electrodes, stimulation parameters, areas being stimulated and recorded, baseline and current signal traces, historical trends in signals, relevant changes in signals, location of signal changes, quality of recorded signals, position of electrodes, alerts due to significant changes in signals, proposed movements to mitigate detrimental signal changes, recorded resultant electrical waveforms, and/or the like. The display 54 may allow an operator to set up the initial monitoring layout and interact with the display 54 during monitoring to add additional information, view information in a different format, and/or respond to alerts. In some implementations, the display 54 may allow override of a change in signal by an anesthesiologist or other medical personnel, etc., such as when a signal change is related to a change in dose of anesthetic agent or some other event unrelated to the stimulation of the nerves of the patient.

FIG. 3 illustrates an example of the display 54, consistent with implementations of the current subject matter. In some implementations, the stimulation system 100 facilitates setup of the stimulation protocol by a clinician and/or non-expert personnel by providing visual cues and instructions during the setup process. For example, as shown in FIG. 3, the display 54 may display pictorial instructions of where to place stimulating and/or recording electrodes, such as the stimulating electrodes 120 and/or the recording electrodes 110, on a patient's body. Such an image may appear at startup of the response identification device 101, upon indicating that monitoring of a new patient is commencing, or upon receiving a signal that a cable has been connected to the response identification device 101. In FIG. 3, each circle represents the recommended location of an electrode.

Generally, the display 54 (e.g., a dynamic display) also improves the manner in which the client device 99 and/or the response identification device 101 displays information and interacts with the user. By dynamically generating values based on an input, the client device 99, and/or the response identification device 101 may reduce the need to render additional complex data entry elements to complete programing. For example, the graphical user interface presented by the display 54 may include graphical elements to increment or decrement a value of the displayed parameters rather than presenting a full keypad for data entry. The client device 99 and/or the response identification device 101 may more efficiently process and validate these input signals, which may be more than entries from freeform text or numeric data entry fields. The use of smaller entry elements also conserves display area on the client device 99 and/or the response identification device 101. This permits presentation of more programming parameters at the time of data entry thereby further reducing the likelihood of a programming error.

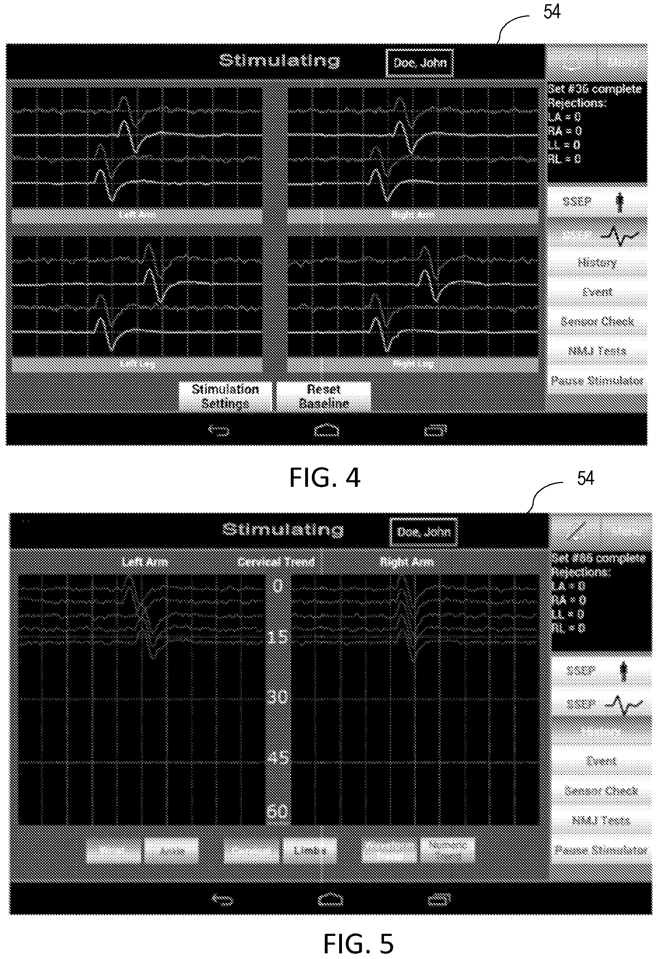

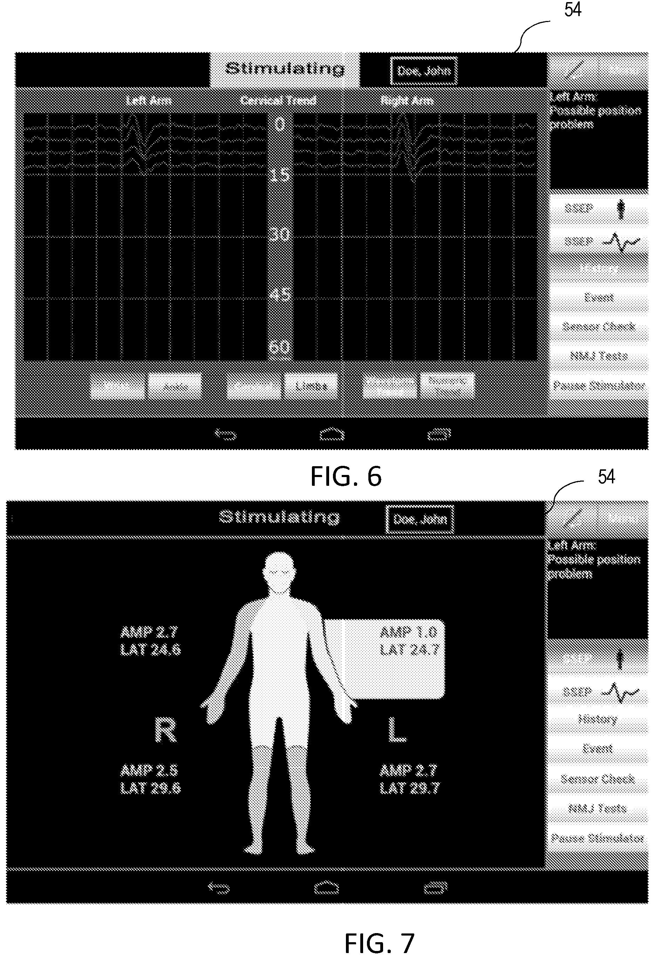

FIGS. 4-9 illustrate examples of the display 54, consistent with implementations of the current subject matter. As depicted in FIG. 4, the waveforms received by the recording electrodes 110 may be presented via the display 54. Additionally and/or alternatively, FIGS. 5 and 6 illustrate examples of the display 54 presenting historical recordings. For example, the provided history spans a particular duration of time, such as 10 minutes, 15 minutes, 30 minutes, 60 minutes, 2 hours, the duration of a surgical procedure, and/or the like. Trends may become visible based on the historical recordings shown by the display 54. For example, an increase in signal latency is visible in the waveform shown by the display 54 in FIG. 5, and a decrease in amplitude is visible in the waveform shown by the display 54 in FIG. 6. Additionally and/or alternatively, the display 54 presents a summary of the acquired data in real time in a pictorial format. For example, as shown in FIG. 7, the display 54 uses colors and/or pictures to indicate whether signals received from a particular limb or body portion are Good, Bad, Undetermined/borderline, or Unreliable. In some implementations, the system 100 automatically determines whether the signals are Good, Bad, Undetermined/borderline, or Unreliable, without real-time monitoring by a user such as an anesthesiologist, nurse, and/or the like, to identify impending peripheral nerve injuries.

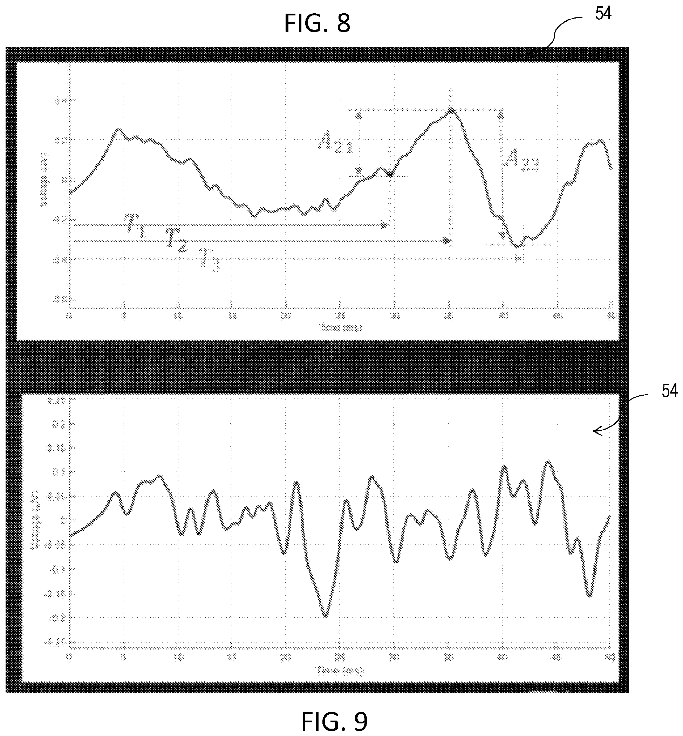

FIGS. 8 and 9 also show examples of the display 54, consistent with implementations of the current subject matter. For example, in FIG. 8, the display 54 displays an example waveform having a positive physiological response. In this example, the waveform is labeled with a positive label, as the illustrated waveform includes a patient physiological response. As shown in FIG. 8, the recorded waveform includes a change in amplitude ($A_{21}$, $A_{23}$) and latency ($T_1$, $T_2$, $T_3$). In FIG. 9, the display 54 displays an example waveform labeled with a negative label. In other words, the example waveform displayed by the display 54 in FIG. 9 does not include a patient physiological response.

Referring back to FIG. 1, the database 125 may include one or more databases, providing physical data storage within a dedicated facility and/or being locally stored on the response identification device 101 and/or the client device 99. Additionally and/or alternatively, the database 125 may include cloud-based systems providing remote storage of data in, for example, a multi-tenant computing environment and/or the like. The database 125 may also include non-transitory computer readable media. The database 125 may store data recorded from and/or calculated based on the waveforms recorded by the recording electrodes 110 and/or received by the response identification device 101. Additionally and/or alternatively, the database 125 may store one or more predicted physiological responses or waveform models generated by the identification controller 102, as described herein.

The database 125 may include and/or be coupled to a server 126, which may be a server coupled to a network, a cloud server, and/or the like. The response identification device 101 and/or the client device 99 may wirelessly communicate with the server 126. The server 126, which may include a cloud-based server, may provide and/or receive data and/or instructions from the data system 125 to the response identification device 101 and/or the client device 99, to implement one or more features of the stimulation system 100, consistent with implementations of the current subject matter. Additionally and/or alternatively, the server 126 may receive data (e.g., one or more waveform signals, patient information, information characterizing the one or more waveform signals, and/or the like) from the response identification device 101 and/or the client device 99.

The identification controller 102 may be at least partially embedded and/or implemented within the response identification device 101 and/or the client 99. The controller 102 may detect and identify, based on the recorded waveforms, a patient physiological response to help prevent or reduce the risk of injury caused to the patient's nerves during surgery.

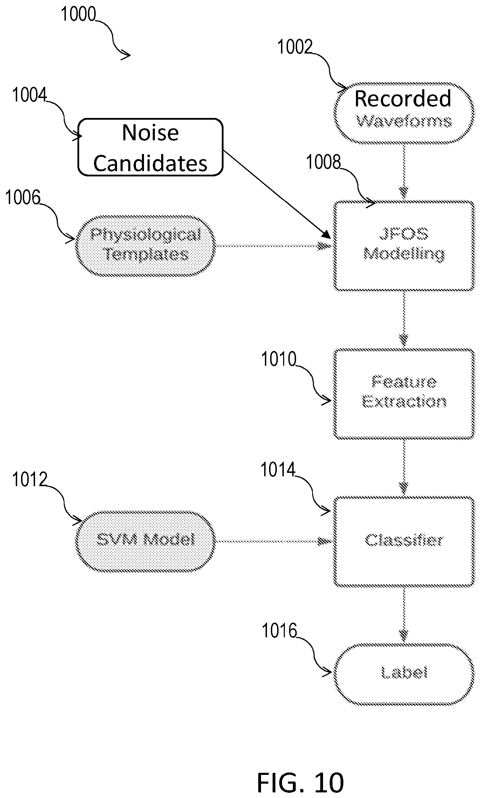

FIG. 10 depicts a flowchart illustrating a method 1000 for identifying and labeling a patient physiological response, consistent with implementations of the current subject matter. During a surgery, or other procedure performed on a patient, the identification controller 102 may cause one or more of the stimulating electrodes 120 to stimulate one or more nerves, such as the tibial (e.g., posterior tibial nerve), saphenous nerve, ulnar nerve, and/or the like, of the patient. In some implementations, the identification controller 102 causes the one or more stimulating electrodes 120 to stimulate the nerves of the patient during surgery, such as during a lumbar surgery.

At 1002, the identification controller 102 may record, via one or more of the recording electrodes 110, a plurality of resultant electrical waveforms, such as a plurality of time-locked resultant electrical waveforms. For example, the recording electrodes 110 may record one, two, three, four, five, six, seven, eight, nine, or ten or more resultant electrical waveforms in response to the delivered electrical stimuli. In some implementations, the recording electrodes 110 detect and/or record the electrical waveforms continuously, at set time intervals (e.g., every 1 second, 15 seconds, 30 seconds, 1 minute, 5 minutes, 15 minutes, 30 minutes, and/or other ranges including ranges therebetween), after each stimulus is delivered to the patient, and/or the like.

Based on the plurality of resultant electrical waveforms, the identification controller 102 may determine whether at least a subset of the plurality of resultant electrical waveforms includes a patient physical response. Determining whether at least the subset of the plurality of resultant electrical waveforms includes the patient physiological response may help to detect whether a surgeon or operating tool is nearing a nerve of the patient, and may help to reduce or prevent injury to the patient during surgery, such as a patient positioning injury. The subset of the plurality of resultant electrical waveforms may include at least two electrical waveforms from the plurality of recorded resultant electrical waveforms. In some implementations, the subset of the plurality of resultant electrical waveforms includes at least two consecutive and/or non-consecutive resultant electrical waveforms. In some implementations, the subset of the plurality of resultant electrical waveforms includes at least three, four, five, six, seven, eight, nine, or ten or more electrical waveforms from the plurality of recorded electrical waveforms.

To determine whether at least the subset of the plurality of resultant electrical waveforms includes the patient physiological response, the identification controller 102 may compare the subset of resultant electrical waveforms of the plurality of resultant electrical waveforms to a model waveform, which may be stored in a database of a plurality of model waveforms. The subset of resultant electrical waveforms may be averaged and then compared to the model waveform and/or each resultant electrical waveform of the subset may be individually compared to the model waveform. Based on this comparison, the identification controller 102 may determine a comparison feature that indicates whether the patient physiological response exists in the subset of resultant electrical waveforms.

In some implementations, the identification controller 102 includes a predicted response model 106 and/or a classification machine learning model 104. At 1008, the predicted response model 106 may generate a model waveform. The model waveform may include and/or be defined by a predicted physiological response and an ensemble average of a plurality of artifact or noise signals. The model waveform may (e.g., automatically) be compared to the subset of resultant electrical waveforms to accurately, quickly, and efficiently identify whether a patient physiological response exists in the subset of resultant electrical waveforms. As noted above, the model waveform may be stored in the database 125. In some implementations, the database 125 stores a plurality of model waveforms. Each of the plurality of model waveforms may be generated by the identification controller 102, such as by the predicted response model 106 for comparison to the subset of resultant electrical waveforms.

The model waveforms may be generated by the predicted response model 106 based on non-patient and/or non-procedure data. In other words, the model waveforms may be generated before a current procedure being performed on the patient, and/or based on data, such as clinical data and/or previously obtained data from previous procedures on the patient or other patients.

The predicted response model 106 may be defined using a joint fast orthogonal search method. In other implementations, other methods may be used, such as fast orthogonal search methods, and/or supervised machine learning topology, among others, to define the predicted response model 106. The predicted response model 106 may be defined using clinical data collected from previous procedures, such as prior lumbar procedures, performed on patients, which include the patient undergoing the current procedure and/or other patients undergoing different procedures. The clinical data may include one or more resultant electrical waveforms from one or more nerves (e.g., the saphenous nerve, the posterior tibial nerve, and/or the like) of the patients and/or one or more corresponding parameters, such as the latencies, amplitudes, quality factors, offset latencies, whether or not a patient physiological response existed, and/or the like. In some implementations, an expert may characterize and/or annotate each resultant electrical waveform collected as part of the clinical data. For example, the expert may characterize and/or annotate each resultant electrical waveform by indicating the ideal placement of markers for onset and peak.

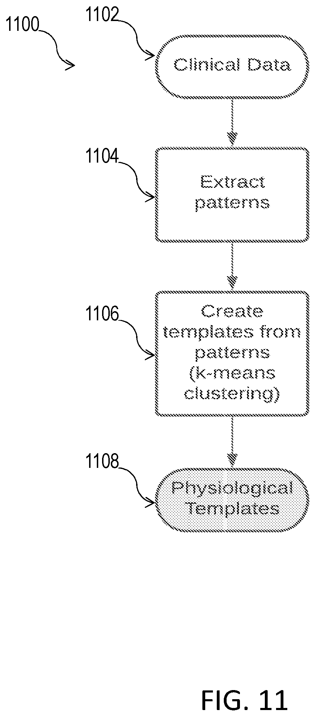

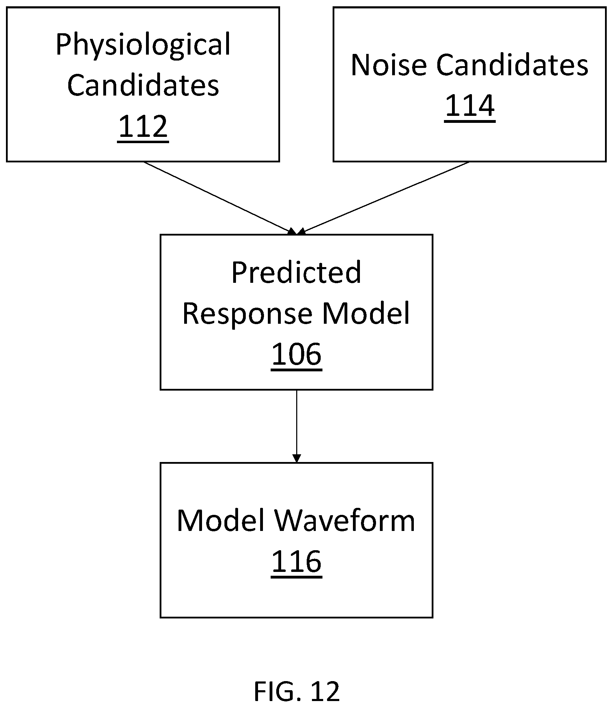

As shown in FIG. 10 at 1004 and 1006, and in FIG. 12, the predicted response model 106 incorporates one or more physiological templates 112 and one or more noise candidates 114. FIG. 11 illustrates an example method 1100 of generating the one or more physiological templates 112, consistent with implementations of the current subject matter. In some implementations, the identification controller 102 may generate one or more physiological templates 112 using the characterized and/or annotated clinical data. In doing so, the identification controller 102 may identify the fewest number of templates that can be representative of all positively labeled physiological responses (e.g., resultant electrical waveforms that are labeled as including the patient physiological response). The physiological templates 112 may form a physiological response candidate pool (e.g., one or more inputs) for use in generating the model waveform 116 by the predicted response model 106, via the joint fast orthogonal search method.

In some implementations, to create the physiological templates, at 1102, the identification controller 102 may receive the clinical data including the resultant electrical waveforms. At 1104, the identification controller 102 may extract one or more patterns from the resultant electrical waveforms. For example, samples before and after the physiological response from each resultant electrical waveform are removed so that only the morphology of interest (e.g., a pattern) remains. At 1106, the patterns are clustered into groups of similar morphology using the k-means clustering method. The pattern with smallest Euclidian distance to the centroid of each cluster is chosen to be a physiological template 112. Each physiological template 112 may be shifted within a wide range of latencies to create a candidate pool for use in generating the model waveform 116. The candidate pool may represent a large population of resultant electrical waveforms. In some implementations, k=8 in the k-means clustering method, which provides a desirable balance between obtaining a unique and diverse set of morphologies for the candidate pool and reducing computing processing requirements. In other implementations, k=1, 2, 3, 4, 5, 6, 7, 8, 9, 10, or more. At 1108, the physiological templates 112 are generated as an output.

As noted above, the predicted response model 106 may be defined using the joint fast orthogonal search method. The joint fast orthogonal search method is an extension of the fast orthogonal search method. While in some instances, the fast orthogonal search method may be used to generate the predicted response model 106, the joint fast orthogonal search method may take into account one or more noise candidates 114, such as a pool of noise and/or artifact signals, when generating the waveform models (e.g., the predicted physiological responses), while the fast orthogonal search method may not. Thus, the joint fast orthogonal search method may generate waveform models that are more accurate and reproducible over time, as it incorporates the noise produced by the main noise sources as described herein. The one or more noise candidates 114 may be generated by incorporating a combination of harmonic noise at 60, 120, and/or 180 Hz, and/or by adding white noise at desired signal to noise ratios.

Additionally and/or alternatively, the joint fast orthogonal search method may minimize the error in the predicted responses over multiple waveforms simultaneously instead of across only one waveform (which is used in the fast orthogonal search method). This configuration also improves the accuracy and reproducibility of the generated waveform models. As shown in FIG. 12, using the joint fast orthogonal search approach, the predicted response model 106 may receive one or more inputs, such as the physiological candidates 112 and the noise candidates 114, and output one or more model waveforms 116 each corresponding to one or more of the following equations, where the equations represent multiple waveform models that may be compared to the resultant electrical waveform or subset of resultant electrical waveforms to determine whether a patient physiological response exists in the subset of the plurality of resultant electrical waveforms:

$$(1)\ \bar{Y}(n) = C_0 + C_1 X_1(n) + \sum_{i=1}^{N} a_i \sin(\omega n + \phi)$$

$$(2)\ \bar{Y}'(n) = C_0' + C_1' X_1(n) + \sum_{i=1}^{N} a_i \sin(\omega n + \phi)$$

$$(3)\ \bar{Y}''(n) = C_0'' + C_1'' X_1(n) + \sum_{i=1}^{N} a_i \sin(\omega n + \phi),$$

where $C_0$ is an arbitrary coefficient, $X_1(n)$ is the selected physiological candidate, $a_i = \sqrt{C_2^2 + C_3^2}$, $\omega$ is the noise frequency, and $\phi$ is an arbitrary phase. As shown in the above equations, the generated waveform model includes both the predicted physiological response and an ensemble average of a plurality of artifact and/or noise signals, which provides more accurate and reproducible results.

Referring to FIG. 10, at 1010, the identification controller 102 may compare the subset of the resultant electrical waveforms to the model waveform. The identification controller 102 may additionally and/or alternatively determine, based on the comparison, one or more comparison features. The comparison feature(s) may indicate whether the patient physiological response exists in the subset of resultant electrical waveforms. Additionally and/or alternatively, the comparison feature(s) may indicate whether an artifact or noise signal exists in the subset of resultant electrical waveforms, and/or whether a portion of the subset of resultant electrical waveforms includes the patient physiological response and/or the artifact signal.

In some implementations, the comparison feature(s) may include one or more of a means squared error between the subset of resultant electrical waveforms and the model waveform, a correlation between the subset of resultant electrical waveforms and the model waveform, a physiological coefficient amplitude, a power of fundamental harmonic noise, a THP of higher harmonic noise, a ratio of a physiological coefficient of the subset of resultant electrical waveforms to a power of the harmonic noise, and a ratio of variance of the patient physiological response of the subset of resultant electrical waveforms to the predicted physiological response of the model. In some implementations, the comparison feature may additionally and/or alternatively represent a comparison of a morphology of the subset of resultant electrical waveforms to a morphology of the model waveform.

As noted above, the comparison feature may include a means squared error between the subset of resultant electrical waveforms and the model waveform. A low means squared error indicates that the subset of resultant electrical waveforms is likely to include the patient physiological response. The low means squared error may include a means squared error that approaches zero, for example, for at least two identical waveforms. A high means squared error indicates that the subset of resultant electrical waveforms is unlikely to include the patient physiological response. The high means squared error may include a means squared error that approaches infinity for at least two different waveforms (e.g., indicating that the at least two waveforms were infinitely different).

In some implementations, the comparison feature(s) includes a ratio of variance of the patient physiological response of the subset of resultant electrical waveforms to the predicted physiological response of the waveform model. A low variance ratio indicates that the subset of resultant electrical waveforms is likely to include the patient physiological response. he low variance ratio may include a variance ratio that approaches zero, for example, for at least two identical waveforms. A high variance ratio indicates that the subset of resultant electrical waveforms is unlikely to include the patient physiological response. The high variance ratio may include a variance ratio that approaches infinity for at least two different waveforms (e.g., indicating that the at least two waveforms were infinitely different).

In some implementations, the comparison feature(s) includes a polarity of each electrical waveform of the subset of resultant electrical waveforms. In some implementations, when each of the electrical waveforms of the subset of resultant electrical waveforms has a same (e.g., positive or negative) polarity, the identification controller 102 may indicate that the subset of resultant electrical waveforms is likely to include the patient physiological response. In some implementations, when at least one of the electrical waveforms of the subset of resultant electrical waveforms has a different (e.g., positive or negative) polarity from the other electrical waveforms of the subset, the identification controller 102 may indicate that the subset of resultant electrical waveforms is not likely to include the patient physiological response.

In some implementations, the identification controller 102 determines that the patient physiological response exists or is likely to exist when the comparison feature of the subset of electrical waveforms is within a threshold range of a threshold value of the comparison feature. The threshold range may be approximately 1 to 5%, 5 to 10%, 10 to 20%, 20 to 30%, and/or other ranges therebetween.

Referring again to FIG. 10, at 1014, the identification controller 102 may classify or label, based on the comparison feature, the subset of resultant electrical waveforms with a positive label or a negative label. Additionally, and/or alternatively, the classification may be based on a mathematical representation of the comparison features, and/or may be based on a comparison between the comparison feature and one or more previously generated comparison features. A positive label may indicate that the patient physiological response is present in the subset of resultant electrical waveforms, while a negative label may indicate that no patient physiological response is present in the subset of resultant electrical waveforms.

At 1012, the identification controller 102 may use the classification machine learning model 104 (also referred to herein as the "classification ML model 104") to classify or label the subset of resultant electrical waveforms. The classification machine learning model 104 may include a support vector machine ("SVM") learning model that is trained for predictive purposes in labeling a subset of resultant electrical waveforms with a positive label and/or a negative label. The classification or labeling may be determined based on the comparison feature. As noted above, labeling the subset of resultant electrical waveforms with a positive label indicates that the patient physiological response exists or is likely to exist in the subset, and labeling the subset of resultant electrical waveforms with a negative label indicates that the patient physiological response does not exist or is not likely to exist in the subset.

In some implementations, the classification ML model 104 may be trained with training data. The training data may include features (e.g., physiological responses, model errors, correlations, and/or other parameters) extracted from model waveforms generated by the predicted response model 106 based on simulated baseline waveforms and the physiological templates 112. The simulated baseline waveforms may be generated by receiving and/or accessing the physiological templates 112. Based on the accessed physiological templates 112, the identification controller 102 may add dispersion to one or more of the physiological templates 112 by expanding or compressing the response onset to offset time to a desired duration, shifting the template peak to a time within an analysis range, adding a combination of harmonic noise, and/or adding white noise at desired signal to noise ratios. This process may be repeated to create a set (e.g., a set of one, two, three, four, five, or more) of simulated baseline waveforms for use in training the classification ML model 104. These simulated baseline waveforms have known labels which form at least a part of the training data used to train the classification ML model 104. Once the classification ML model 104 is trained, the trained classification ML model 104 may classify or label, at 1016, at least the subset of resultant electrical waveforms with a positive or negative label to indicate whether the patient physiological response exists or is likely to exist within the subset of resultant electrical waveforms.

In some implementations, the identification controller 102 may cause the display 54 to display, and the display 54 may display, an indication that the patient physiological response exists in the subset of resultant electrical waveforms, such as when the classification ML model 104 labels the subset of resultant electrical waveforms with a positive label. Additionally and/or alternatively, the identification controller 102 may cause the display 54 to display, and the display 54 may display, an indication that the patient physiological response does not exist in the subset of resultant electrical waveforms or that only an artifact or noise signal exists in the subset of resultant electrical waveforms, such as when the classification ML model 104 labels the subset of resultant electrical waveforms with a negative label. Thus, the stimulation system 100 may automatically, accurately, and effectively identify and determine whether recorded waveforms include the patient physiological response to help reduce or eliminate the risk of causing an injury to the patient during surgery or another procedure.

In some implementations, a method of performing surgery or other procedure as described herein includes performing a robotically-assisted surgical procedure, such as, for example, a robotically-assisted hysterectomy, other gynecologic surgical procedure, prostatectomy, urologic surgical procedure, general laparoscopic surgical procedure, thoracoscopic surgical procedure, valve replacement, other cardiac surgical procedure, bariatric surgery, other gastrointestinal surgical procedure, or oncological surgical procedures, among others. The method of some implementations further includes delivering an electrical stimulus to a peripheral nerve in the body, recording a resultant electrical waveform generated by the body's nervous system in response to the electrical stimulus, and monitoring the resultant electrical waveform to detect changes indicative of potential nerve injury. Additionally or alternatively, in some implementations, the method of performing surgery may include any of the methods for detecting the functionality of one or more nerves described elsewhere herein. The methods of detecting functionality of one or more nerves or of using the response identification device 101 may be incorporated at any juncture of a robotic surgery. For example, such methods can be performed at multiple times, continuously, at pre-selected situations such as when certain types of procedures are initiated or concluded (including any of those mentioned above), and so forth. The method of various implementations further includes adjusting the position of a patient when a potential nerve injury or abnormality is detected.

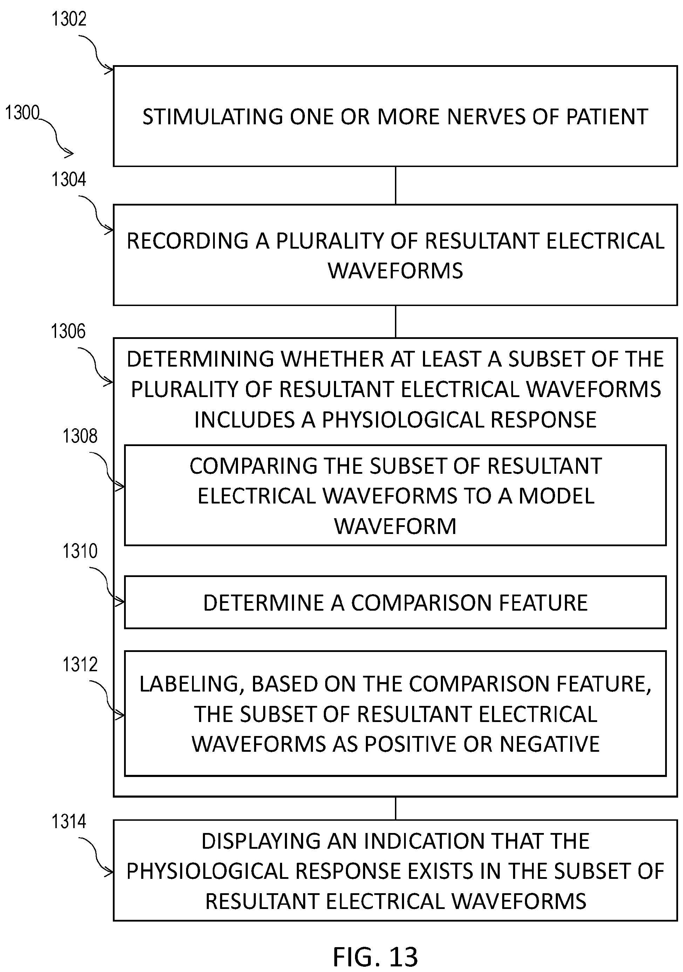

FIG. 13 depicts a method 1300 for identifying and labeling a patient physiological response, consistent with implementations of the current subject.

At 1302, the system (e.g., via the identification controller 102) may stimulate, via a stimulating electrode coupled to a patient, one or more nerves of the patient. For example, the system may stimulate a tibial nerve (e.g., posterior tibial nerve), saphenous nerve, the ulnar nerve, and/or the like, of the patient during a surgery or other procedure. The stimulating electrode may include one or more electrodes in communication with a response identification device (e.g., the response identification device 101), which is configured to monitor the one or more nerves of the patient. In some implementations, the stimulation is delivered to the patient continuously, at various time intervals, according to a stimulation protocol, and/or the like. The stimulation may include transmission of a plurality of electrical stimuli.

At 1304, the system, such as via the identification controller 102, may record, via a recording electrode coupled to the patient, a plurality of resultant electrical waveforms. The plurality of resultant electrical waveforms may include one, two, three, four, five, or more resultant electrical waveforms. The plurality of resultant electrical waveforms may be received by the response identification device. The resultant electrical waveforms may be generated by the patient in response to the delivered electrical stimuli.

At 1306, the system may determine whether at least a subset of the plurality of resultant electrical waveforms includes a physiological response. This determination may help to prevent injury to the patient during surgery or another operation. For example, at 1308, the subset of resultant electrical waveforms of the plurality of resultant electrical waveforms may be compared to a model waveform from a database of a plurality of model waveforms. The model waveforms may include a predicted physiological response and an ensemble average of a plurality of artifact or noise signals. The model waveform may be generated (such as by the predicted response model 106) using a joint fast orthogonal search method, as described herein, that incorporates both physiological templates (e.g., predicted physiological responses) and artifact or noise candidates.

As described herein, the plurality of model waveforms may be generated based on clinical data including non-procedure or non-patient information. In other words, the plurality of template waveforms stored in the database may be generated based on information collected from procedures and/or patients that are not the current procedure and/or the current patient. The database may also store the clinical data and/or be loaded with the clinical data collected prior to the current operation and not based on the operation the patient is currently undergoing.

At 1310, a comparison feature may be determined based on the comparison. The comparison feature may indicate whether the patient physiological response exists in the subset of resultant electrical waveforms. The comparison feature may additionally and/or alternatively indicate whether the subset of resultant electrical waveforms includes only an artifact or noise signal. In some implementations, the comparison feature(s) comprises one or more of a means squared error between the subset of resultant electrical waveforms and the model waveform, a correlation between the subset of resultant electrical waveforms and the model waveform, a physiological coefficient amplitude, a power of fundamental harmonic noise, a THP of higher harmonic noise, a ratio of a physiological coefficient of the subset of resultant electrical waveforms to a power of the harmonic noise, and a ratio of variance of the patient physiological response of the subset of resultant electrical waveforms to the predicted physiological response of the model, and/or the like. Additionally and/or alternatively, the comparison feature represents a comparison of a morphology of the subset of resultant electrical waveforms to a morphology of the model waveform. The comparison feature may include a plurality of comparison features.

At 1312, the subset of resultant electrical waveforms may be labeled, based on at least the comparison feature, as positive or negative. A positive label indicates that a patient physiological response is present or is likely to be present in the subset of recorded electrical waveforms. A negative label indicates that a patient physiological response is not present or is not likely to be present in the subset of recorded electrical waveforms. The labeling and/or classification of the subset of resultant electrical waveforms may be performed by a machine learning model (e.g., a support vector machine learning model), such as the trained classification ML model 104 described herein.

At 1314, an indication that the physiological response exists in the subset of resultant electrical waveforms may be displayed on a display (e.g., the display 54). The display may be coupled to the response identification device 101. The indication may include one or more alerts, such as one or more audio, visual, and/or tactile alerts or signals. The indication may indicate that the surgeon is approaching a nerve of a patient. In some implementations, the indication additionally and/or alternatively indicates that the subset of resultant electrical waveforms does not include the physiological response or includes only noise or artifact signals. Thus, the stimulation system 100 may automatically, accurately, and effectively identify and determine whether recorded waveforms include the patient physiological response to help reduce or eliminate the risk of causing an injury to the patient during surgery or another procedure.

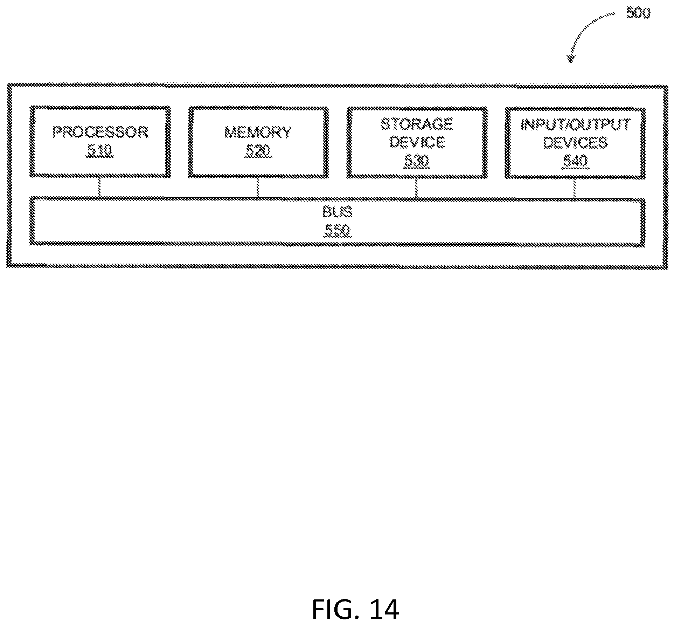

FIG. 14 depicts a block diagram illustrating a computing system 500 consistent with implementations of the current subject matter. Referring to FIGS. 1 and 14, the computing system 500 can be used to implement the stimulation system 100 and/or any components therein.

As shown in FIG. 14, the computing system 500 can include a processor 510, a memory 520, a storage device 530, and input/output devices 540. The processor 510, the memory 520, the storage device 530, and the input/output devices 540 can be interconnected via a system bus 550. The processor 510 is capable of processing instructions for execution within the computing system 500. Such executed instructions can implement one or more components of, for example, the configuration engine 110. In some example implementations, the processor 510 can be a single-threaded processor. Alternatively, the processor 510 can be a multi-threaded processor. The processor 510 is capable of processing instructions stored in the memory 520 and/or on the storage device 530 to present graphical information for a user interface provided via the input/output device 540.

The memory 520 is a computer readable medium such as volatile or nonvolatile that stores information within the computing system 500. The memory 520 can store data structures representing configuration object databases, for example. The storage device 530 is capable of providing persistent storage for the computing system 500. The storage device 530 can be a floppy disk device, a hard disk device, an optical disk device, or a tape device, or other suitable persistent storage means. The input/output device 540 provides input/output operations for the computing system 500. In some example implementations, the input/output device 540 includes a keyboard and/or pointing device. In various implementations, the input/output device 540 includes a display unit for displaying graphical user interfaces.

According to some example implementations, the input/output device 540 can provide input/output operations for a network device. For example, the input/output device 540 can include Ethernet ports or other networking ports to communicate with one or more wired and/or wireless networks (e.g., a local area network (LAN), a wide area network (WAN), the Internet).

In some example implementations, the computing system 500 can be used to execute various interactive computer software applications that can be used for organization, analysis and/or storage of data in various formats. Alternatively, the computing system 500 can be used to execute software applications. These applications can be used to perform various functionalities, e.g., planning functionalities (e.g., generating, managing, editing of spreadsheet documents, word processing documents, and/or any other objects, etc.), computing functionalities, communications functionalities, etc. The applications can include various add-in functionalities or can be standalone computing products and/or functionalities. Upon activation within the applications, the functionalities can be used to generate the user interface provided via the input/output device 540. The user interface can be generated and presented to a user by the computing system 500 (e.g., on a computer screen monitor, etc.).

One or more aspects or features of the subject matter described herein can be realized in digital electronic circuitry, integrated circuitry, specially designed ASICs, field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are remote from each other and typically interact through a communication network. The relationship of client and server arises by virtue of computer programs running on the respective computers and having a client-server relationship to each other.

These computer programs, which can also be referred to as programs, software, software applications, applications, components, or code, include machine instructions for a programmable processor, and can be implemented in a high-level procedural and/or object-oriented programming language, and/or in assembly/machine language. As used herein, the term "machine-readable medium" refers to any computer program product, apparatus, and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example, as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and one or more hardware buttons, a keyboard and/or a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including acoustic, speech, or tactile input. Other possible input devices include touch screens or other touch-sensitive devices such as single or multi-point resistive or capacitive track pads, voice recognition hardware and software, optical scanners, optical pointers, digital image capture devices, hardware buttons, and associated interpretation software, and the like.

Although the disclosure, including the figures, described herein may describe and/or exemplify different variations separately, it should be understood that all or some, or components of them, may be combined.

Although various illustrative implementations are described above, any of a number of changes may be made to various implementations. For example, the order in which various described method steps are performed may often be changed in alternative implementations, and in other alternative implementations one or more method steps may be skipped altogether. Optional features of various device and system implementations may be included in some implementations and not in others. Therefore, the foregoing description is provided primarily for exemplary purposes and should not be interpreted to limit the scope of the claims.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other implementations. References to a structure or feature that is disposed "adjacent" another feature may have portions that overlap or underlie the adjacent feature.

Terminology used herein is for the purpose of describing particular implementations only and is not intended to be limiting. For example, as used herein, the singular forms "a," "an," and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as, for example, "under," "below," "lower," "over," "upper," and the like, may be used herein for ease of description to describe one element or one feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly," "downwardly," "vertical," "horizontal," and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings provided herein.

Throughout this specification and the claims which follow, unless the context requires otherwise, the word "comprise" and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" "or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise.

The examples and illustrations included herein show, by way of illustration and not of limitation, specific implementations in which the subject matter may be practiced. As mentioned, other implementations may be utilized and derived there from, such that structural and logical substitutions and changes may be made without departing from the scope of this disclosure. Although specific implementations have been illustrated and described herein, any arrangement calculated to achieve the same purpose may be substituted for the specific implementations shown. This disclosure is intended to cover any and all adaptations or variations of various implementations. Combinations of the above implementations, and other implementations not specifically described herein, are possible.

In the descriptions above and in the claims, phrases such as, for example, "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH™, JAVA™, .NET™, C, C++, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

The examples and illustrations included herein show, by way of illustration and not of limitation, specific implementations in which the subject matter may be practiced. As mentioned, other implementations may be utilized and derived there from, such that structural and logical substitutions and changes may be made without departing from the scope of this disclosure. Such implementations of the inventive subject matter may be referred to herein individually or collectively by the term "invention" merely for convenience and without intending to voluntarily limit the scope of this application to any single invention or inventive concept, if more than one is, in fact, disclosed. Thus, although specific implementations have been illustrated and described herein, any arrangement calculated to achieve the same purpose may be substituted for the specific implementations shown. This disclosure is intended to cover any and all adaptations or variations of various implementations. Combinations of the above implementations, and other implementations not specifically described herein, will be apparent to those of skill in the art upon reviewing the above description.

## Claims

1. A stimulation system (100) for detecting and identifying a patient physiological response, the stimulation system comprising:

   at least one processor (510); and
   at least one memory (520) storing instructions which, when executed by the at least one data processor, result in operations comprising:

stimulating, via a stimulating electrode (120) coupled to a patient (10), one or more nerves of the patient;
recording, via a recording electrode (110) coupled to the patient, a plurality of resultant electrical waveforms;
determining, based on the plurality of resultant electrical waveforms, whether at least a subset of the plurality of resultant electrical waveforms includes a patient physiological response, the determining comprising:

comparing the subset of resultant electrical waveforms of the plurality of resultant electrical waveforms to a model waveform (116) derived from a database of a plurality of template waveforms (112, 114); and
determining, based on the comparison, a comparison feature, the comparison feature indicating whether the patient physiological response exists in the subset of resultant electrical waveforms; and

displaying, via a display (54), an indication when the patient physiological response exists in the subset of resultant electrical waveforms.

2. The system (100) of claim 1, wherein the model waveform (116) comprises a predicted physiological response and an ensemble average of a plurality of artifact signals.

3. The system (100) of claim 2, wherein the comparison feature further indicates whether an artifact signal exists in the subset of resultant electrical waveforms, the artifact signal generated by noise.

4. The system (100) of claim 3, wherein the operations further comprise: displaying, via the display (54), an indication that the artifact signal exists in the subset of resultant electrical waveforms.

5. The system (100) of any one of claims 1 to 4, wherein the determining whether at least the subset of the plurality of resultant electrical waveforms includes the patient physiological response further comprises: labeling, based on the comparison feature, the subset of the plurality of resultant electrical waveforms with a positive label or a negative label, the positive label indicating that the patient physiological response exists, and the negative label indicating that the patient physiological response does not exist.

6. The system (100) of any one of claims 1 to 5, wherein the determination of whether the plurality of resultant electrical waveforms includes the patient physiological response further comprises: determining that the patient physiological response exists when the comparison feature of the subset of electrical waveforms is within a threshold range of a threshold value of the comparison feature.

7. The system (100) of claim 6, wherein the comparison feature comprises one or more of a means squared error between the subset of resultant electrical waveforms and the model waveform (116), a correlation between the subset of resultant electrical waveforms and the model waveform (116), a physiological coefficient amplitude, a power of fundamental harmonic noise, a THP of higher harmonic noise, a ratio of a physiological coefficient of the subset of resultant electrical waveforms to a power of the harmonic noise, and a ratio of variance of the patient physiological response of the subset of resultant electrical waveforms to the predicted physiological response of the model.

8. The system of any one of claims 1 to 7, wherein the determination of whether the plurality of resultant electrical waveforms includes the patient physiological response further comprises: determining that the patient physiological response exists when a polarity of each electrical waveform of the subset of resultant electrical waveforms is the same.

9. The system of any one of claims 1 to 8, wherein the comparison feature represents a comparison of a morphology of the subset of resultant electrical waveforms to a morphology of the model waveform (116).

10. The system of claim 5, wherein the comparison feature comprises a plurality of comparison features, and wherein the labeling is further based on a mathematical representation of the plurality of comparison features.

11. The system of claim 5, wherein the labeling comprises comparing the comparison feature to a plurality of previously generated comparison features.

12. The system of any of claims 1 to 11, wherein the subset of the plurality of resultant electrical waveforms are time-locked.

13. The system of any one of claims 1 to 12, wherein the stimulating comprises transmitting a plurality of electrical stimuli;

and wherein the stimulating electrode is in communication with an evoked potential detection device (101) configured to monitor the one or more peripheral nerves of the patient.

14. The system (100) of claim 13, wherein the plurality of resultant electrical waveforms is received by the evoked potential detection device (101), the resultant electrical waveforms generated by the patient in response to the electrical stimuli.

15. The system (100) of any one of claims 1 to 14, wherein the template waveforms (112, 114) comprise at least one physiological candidate (112) and at least one noise candidate (114).

**Patentansprüche**

1. Stimulationssystem (100) zum Erkennen und Identifizieren einer physiologischen Reaktion eines Patienten, wobei das Stimulationssystem umfasst:

   mindestens einen Prozessor (510); und
   mindestens einen Speicher (520), in dem Anweisungen gespeichert sind, die, wenn sie durch den mindestens einen Datenprozessor ausgeführt werden, zu Operationen führen, die umfassen:

   Stimulieren, über eine Stimulationselektrode (120), die mit einem Patienten (10) verbunden ist, eines oder mehrerer Nerven des Patienten;
   Aufzeichnen, über eine Aufzeichnungselektrode (110), die mit dem Patienten verbunden ist, mehrerer resultierender elektrischer Wellenformen;
   Bestimmen, basierend auf den mehreren resultierenden elektrischen Wellenformen, ob mindestens eine Teilmenge der mehreren resultierenden elektrischen Wellenformen eine physiologische Reaktion des Patienten beinhaltet, wobei das Bestimmen umfasst:

      Vergleichen der Teilmenge der resultierenden elektrischen Wellenformen der mehreren resultierenden elektrischen Wellenformen mit einer Modellwellenform (116), die aus einer Datenbank mit mehreren Vorlagenwellenformen (112, 114) abgeleitet wurde; und
      Bestimmen, basierend auf dem Vergleich, eines Vergleichsmerkmals, wobei das Vergleichsmerkmal anzeigt, ob die physiologische Reaktion des Patienten in der Teilmenge der resultierenden elektrischen Wellenformen vorhanden ist; und
      Anzeigen, über ein Display (54), einer Anzeige, wenn die physiologische Reaktion des Patienten in der Teilmenge der resultierenden elektrischen Wellenformen vorhanden ist.

2. System (100) nach Anspruch 1, wobei die Modellwellenform (116) eine vorhergesagte physiologische Reaktion und einen Ensemble-Mittelwert mehrerer Artefaktsignale umfasst.

3. System (100) nach Anspruch 2, wobei das Vergleichsmerkmal ferner anzeigt, ob ein Artefaktsignal in der Teilmenge der resultierenden elektrischen Wellenformen vorhanden ist, wobei das Artefaktsignal durch Rauschen erzeugt wird.

4. System (100) nach Anspruch 3, wobei die Operationen ferner umfassen: Anzeigen, über das Display (54), einer Anzeige, dass das Artefaktsignal in der Teilmenge der resultierenden elektrischen Wellenformen vorhanden ist.

5. System (100) nach einem der Ansprüche 1 bis 4, wobei das Bestimmen, ob zumindest die Teilmenge der mehreren resultierenden elektrischen Wellenformen die physiologische Reaktion des Patienten beinhaltet, ferner umfasst: Kennzeichnen, basierend auf dem Vergleichsmerkmal, der Teilmenge der mehreren resultierenden elektrischen Wellenformen mit einer positiven Kennzeichnung oder einer negativen Kennzeichnung, wobei die positive Kennzeichnung anzeigt, dass die physiologische Reaktion des Patienten vorhanden ist, und die negative Kennzeichnung anzeigt, dass die physiologische Reaktion des Patienten nicht vorhanden ist.

6. System (100) nach einem der Ansprüche 1 bis 5, wobei die Bestimmung, ob die mehreren resultierenden elektrischen Wellenformen die physiologische Reaktion des Patienten beinhalten, ferner umfasst: Bestimmen, dass die physiologische Reaktion des Patienten vorhanden ist, wenn das Vergleichsmerkmal der Teilmenge der elektrischen Wellenformen innerhalb eines Schwellenbereichs eines Schwellenwerts des Vergleichsmerkmals liegt.

7. System (100) nach Anspruch 6, wobei das Vergleichsmerkmal eines oder mehrere der folgenden umfasst: einen mittleren quadratischen Fehler zwischen der Teilmenge der resultierenden elektrischen Wellenformen und der Modellwellenform (116), eine Korrelation zwischen der Teilmenge der resultierenden elektrischen Wellenformen und der Modellwellenform (116), eine physiologische Koeffizientenamplitude, eine Potenz des harmonischen Grundrauschens, eine THP des harmonischen Grundrauschens, ein Verhältnis eines physiologischen Koeffizienten der Teilmenge der resultierenden elektrischen Wellenformen zu einer Potenz des harmonischen Rauschens und ein Verhältnis der Varianz der physiologischen Reaktion des Patienten der Teilmenge der resultierenden elektrischen Wellenformen zu der vorhergesagten physiologischen Reaktion des Modells.

8. System nach einem der Ansprüche 1 bis 7, wobei die Bestimmung, ob die mehreren resultierenden elektrischen Wellenformen die physiologische Reaktion des Patienten beinhalten, ferner umfasst: Bestimmen, dass die physiologische Reaktion des Patienten vorhanden ist, wenn eine Polarität jeder elektrischen Wellenform der Teilmenge der resultierenden elektrischen Wellenformen gleich ist.

9. System nach einem der Ansprüche 1 bis 8, wobei das Vergleichsmerkmal einen Vergleich einer Morphologie der Teilmenge der resultierenden elektrischen Wellenformen mit einer Morphologie der Modellwellenform (116) darstellt.

10. System nach Anspruch 5, wobei das Vergleichsmerkmal mehrere Vergleichsmerkmale umfasst und wobei das Kennzeichnen ferner auf einer mathematischen Darstellung der mehreren Vergleichsmerkmale basiert.

11. System nach Anspruch 5, wobei das Kennzeichnen das Vergleichen des Vergleichsmerkmals mit mehreren zuvor erzeugten Vergleichsmerkmalen umfasst.

12. System nach einem der Ansprüche 1 bis 11, wobei die Teilmenge der mehreren resultierenden elektrischen Wellenformen zeitlich gekoppelt ist.

13. System nach einem der Ansprüche 1 bis 12, wobei das Stimulieren das Übertragen mehrerer elektrischer Reize umfasst und wobei die Stimulationselektrode mit einer Vorrichtung (101) zur Erkennung evozierter Potenziale in Kommunikationsverbindung steht, die zur Überwachung eines oder mehrerer peripherer Nerven des Patienten konfiguriert ist.

14. System (100) nach Anspruch 13, wobei die mehreren resultierenden elektrischen Wellenformen durch die Vorrichtung zur Erkennung evozierter Potenziale (101) empfangen werden, wobei die resultierenden elektrischen Wellenformen durch den Patienten als Reaktion auf die elektrischen Reize erzeugt werden.

15. System (100) nach einem der Ansprüche 1 bis 14, wobei die Vorlagenwellenformen (112, 114) mindestens einen physiologischen Kandidaten (112) und mindestens einen Rauschkandidaten (114) umfassen.

**Revendications**

1. Système (100) de stimulation pour détecter et identifier une réponse physiologique du patient, le système de stimulation comportant :

   au moins un processeur (510) ; et
   au moins une mémoire (520) stockant des instructions qui, lorsqu'elles sont exécutées par l'au moins un processeur de données, donnent des opérations comprenant :

      la stimulation, par le biais d'une électrode de stimulation (120) couplée à un patient (10), d'un ou de plusieurs nerfs du patient ;
      l'enregistrement, par le biais d'une électrode d'enregistrement (110) couplée au patient, d'une pluralité de formes d'ondes électriques résultantes ;
      la détermination, sur la base de la pluralité de formes d'ondes électriques résultantes, établissant si au moins un sous-ensemble de la pluralité de formes d'ondes électriques résultantes comporte ou non une réponse physiologique du patient, la détermination comprenant :

         la comparaison du sous-ensemble de formes d'onde électriques résultantes de la pluralité de formes d'onde électriques résultantes à une forme d'onde modèle (116) dérivée d'une base de données d'une pluralité de formes d'onde types (112, 114) ; et

la détermination, sur la base de la comparaison, d'une caractéristique de comparaison, la caractéristique de comparaison indiquant si la réponse physiologique du patient existe dans le sous-ensemble de formes d'ondes électriques résultantes ; et

l'affichage, par l'intermédiaire d'un afficheur (54), d'une indication lorsque la réponse physiologique du patient existe dans le sous-ensemble de formes d'ondes électriques résultantes.

2. Système (100) selon la revendication 1, dans lequel la forme d'onde modèle (116) comprend une réponse physiologique prédite et une moyenne d'ensemble d'une pluralité de signaux d'artefact.

3. Système (100) selon la revendication 2, dans lequel la caractéristique de comparaison indique en outre si un signal d'artefact existe dans le sous-ensemble de formes d'onde électriques résultantes, le signal d'artefact étant généré par du bruit.

4. Système (100) selon la revendication 3, dans lequel les opérations comprennent en outre : l'affichage, par le biais de l'afficheur (54), d'une indication selon laquelle le signal d'artefact existe dans le sous-ensemble de formes d'onde électriques résultantes.

5. Système (100) selon l'une quelconque des revendications 1 à 4, dans lequel la détermination établissant si au moins le sous-ensemble de la pluralité de formes d'ondes électriques résultantes comporte la réponse physiologique du patient comprend en outre : l'étiquetage, sur la base de la caractéristique de comparaison, du sous-ensemble de la pluralité de formes d'ondes électriques résultantes avec une étiquette positive ou une étiquette négative, l'étiquette positive indiquant que la réponse physiologique du patient existe, et l'étiquette négative indiquant que la réponse physiologique du patient n'existe pas.

6. Système (100) selon l'une quelconque des revendications 1 à 5, dans lequel la détermination établissant si la pluralité de formes d'ondes électriques résultantes comporte ou non la réponse physiologique du patient comprend en outre : la détermination que la réponse physiologique du patient existe lorsque la caractéristique de comparaison du sous-ensemble de formes d'ondes électriques se trouve dans une plage de seuil d'une valeur de seuil de la caractéristique de comparaison.

7. Système (100) selon la revendication 6, dans lequel la caractéristique de comparaison comprend un ou plusieurs d'une erreur quadratique moyenne entre le sous-ensemble de formes d'onde électriques résultantes et la forme d'onde modèle (116), d'une corrélation entre le sous-ensemble de formes d'onde électriques résultantes et la forme d'onde modèle (116), d'une amplitude de coefficient physiologique, d'une puissance de bruit harmonique fondamental, d'un THP de bruit harmonique supérieur, d'un rapport entre un coefficient physiologique du sous-ensemble de formes d'ondes électriques résultantes et une puissance du bruit harmonique, et d'un rapport entre la variance de la réponse physiologique du patient du sous-ensemble de formes d'ondes électriques résultantes et la réponse physiologique prédite du modèle.

8. Système selon l'une quelconque des revendications 1 à 7, dans lequel la détermination établissant si la pluralité de formes d'onde électriques résultantes comporte la réponse physiologique du patient comprend en outre : la détermination que la réponse physiologique du patient existe lorsqu'une polarité de chaque forme d'onde électrique du sous-ensemble de formes d'onde électriques résultantes est la même.

9. Système selon l'une quelconque des revendications 1 à 8, dans lequel la caractéristique de comparaison représente une comparaison d'une morphologie du sous-ensemble de formes d'onde électriques résultantes à une morphologie de la forme d'onde modèle (116).

10. Système selon la revendication 5, dans lequel la caractéristique de comparaison comprend une pluralité de caractéristiques de comparaison, et dans lequel l'étiquetage est en outre basé sur une représentation mathématique de la pluralité de caractéristiques de comparaison.

11. Système selon la revendication 5, dans lequel l'étiquetage comprend la comparaison de la caractéristique de comparaison à une pluralité de caractéristiques de comparaison générées précédemment.

12. Système selon l'une quelconque des revendications 1 à 11, dans lequel le sous-ensemble de la pluralité de formes d'onde électriques résultantes est verrouillé dans le temps.

13. Système selon l'une quelconque des revendications 1 à 12, dans lequel la stimulation comprend la transmission d'une pluralité de stimuli électriques ; et dans lequel l'électrode de stimulation est en communication avec un dispositif de détection de potentiel évoqué (101) configuré pour surveiller le ou les nerfs périphériques du patient.

14. Système (100) selon la revendication 13, dans lequel la pluralité de formes d'onde électriques résultantes est reçue par le dispositif de détection de potentiel évoqué (101), les formes d'onde électriques résultantes étant générées par le patient en réponse aux stimuli électriques.

15. Système (100) selon l'une quelconque des revendications 1 à 14, dans lequel les formes d'onde modèles (112, 114) comprennent au moins un candidat physiologique (112) et au moins un candidat bruit (114).

Database
125

Server 126

Response
Identification Device
101

NETWORK
150

100

Client Device
99

Display
54

Identification Controller
102

Classification
ML Model
104

Predicted
Response
Model
106

FIG. 1

FIG. 2

FIG. 3

54

FIG. 4

54

FIG. 5

FIG. 6

FIG. 7

FIG. 8

FIG. 9

FIG. 10

1100

1102 Clinical Data

1104 Extract patterns

1106 Create templates from patterns (k-means clustering)

1108 Physiological Templates

FIG. 11

Physiological Candidates
112

Noise Candidates
114

Predicted Response Model
106

Model Waveform
116

FIG. 12

1302

1300

STIMULATING ONE OR MORE NERVES OF PATIENT

1304

RECORDING A PLURALITY OF RESULTANT ELECTRICAL WAVEFORMS

1306

DETERMINING WHETHER AT LEAST A SUBSET OF THE PLURALITY OF RESULTANT ELECTRICAL WAVEFORMS INCLUDES A PHYSIOLOGICAL RESPONSE

1308

COMPARING THE SUBSET OF RESULTANT ELECTRICAL WAVEFORMS TO A MODEL WAVEFORM

1310

DETERMINE A COMPARISON FEATURE

1312

LABELING, BASED ON THE COMPARISON FEATURE, THE SUBSET OF RESULTANT ELECTRICAL WAVEFORMS AS POSITIVE OR NEGATIVE

1314

DISPLAYING AN INDICATION THAT THE PHYSIOLOGICAL RESPONSE EXISTS IN THE SUBSET OF RESULTANT ELECTRICAL WAVEFORMS

FIG. 13

500

| PROCESSOR 510 | MEMORY 520 | STORAGE DEVICE 530 | INPUT/OUTPUT DEVICES 540 |

BUS
550

# FIG. 14

**EP 4 304 705 B1**

**Patent documents cited in the description**

- US 63160605

- US 20140324118 A1